# EUROPEAN PATENT SPECIFICATION

(11) **EP 2 497 533 B1**
(45) Date of publication and mention of the grant of the patent: **13.11.2013**
(21) Application number: 12158664.8
(22) Date of filing: 08.03.2012
(51) Int. Cl.: A61N 5/06, A61N 1/32

(54) **Device for treating cellulite**
Vorrichtung zur Behandlung von Zellulitis
Dispositif de traitement de la cellulite

(30) Priority: 11.03.2011 ES 201130340
(43) Date of publication of application: 12.09.2012
(73) Proprietor: Arriaza Muñoz, Francisco José, 08203 Sabadell (ES)
(72) Inventor: Arriaza Muñoz, Francisco José, 08203 Sabadell (ES)
(74) Representative: Isern-Jara, Nuria

(56) References cited:
- EP-A1- 1 905 409
- US-A1- 2010 049 177

## Description

An object of this invention is a device for the treatment of cellulite, used in a non-invasive technique to remove localized build-ups of fat rapidly, safely and long-lastingly. This invention comprises a device that uses a specific combination of laser and electric currents favouring the removal of this fat, generating a process that breaks down the triglycerides stored in fatty acids and glycerol in the adipocytes, increasing the permeability of their walls and releasing them through the membrane pores, in such a way that they enter circulation through the blood and lymphatic system, being turned into fuel, and helping them to disappear through daily exercise.

### PRIOR ART

Cellulite is commonly known as a build-up of adipose tissue in certain parts of the body, forming adipose nodules of fat, water and toxins. Other names for this entity are: gynoid lipodystrophy, Orange Peel Syndrome or the "mattress" phenomenon. Between 85% and 98% of women show some signs of cellulite after puberty, it being more common in some ethnic groups than in others. There would appear to be a hormonal element in its development; cellulite is rarely found in men.

There are several degrees of cellulite, soft cellulite, hard cellulite and the best known one, which is sclerotic cellulite (orange peel syndrome). The clinical picture of cellulite is the result of an alteration in the circulation of the fat layer (hypodermis): the fat layer grows and the side walls swell to form dimples; that is why physical exercise such as running is important, to strengthen the muscles of the glutei and thighs.

The fat cells are naturally arranged in the genetically predisposed zones. The function of this mechanism, called lipogenesis, is to store the reserve fat for the organism. A sedentary life prevents this fat reserve from being sufficiently used, and lipolysis, the mechanism for using or releasing fat, gradually stops carrying out its activity.

The state of the art includes well-known treatments against cellulite that on the one hand, use electric currents and, on the other hand, involve treatment with soft laser. However, the use of laser emitters in the form of a longitudinal beam like the ones used until the present time (spot beams) and the tropicalisation in the circuits, more specifically, covering the high-frequency electrodes with insulating material and submerging the rear part of the diodes in resin, keeping the emitting head slotted into an aluminium dissipater, which is also with its rear part bathed in resin, makes combined use possible without any spurious jumps or interference, which had made the two techniques incompatible until the present time.

For example, in the patent application EP1905409 is disclosed an apparatus for reactivating microcirculation of the blood, wherein the main feature of this apparatus is that has a heartbeat detecting device and an electronic central control unit for controlling the valve to activate the suction stage in time with the contraction of cardiac muscle. This apparatus has a first handset comprising a disk, with a skin-contacting face which is fitted with bodies contacting the skin and actuating means for operating said disk so that said bodies massage the skin. This invention provides a mechanical stimulation of the skin. Additionally, it mentions the use of electrodes by electrically stimulation and relaxation of muscle tissue and the use of laser beam transmitters which enhances vascularisation and cell metabolism.

The above document does not disclosed the requirements to apply a combination of laser beam and current emitter electrodes for the treatment of cellulites, in such way that will be possible to stimulate the lipolisis and increase the permeability of the adipocytes, favouring the expulsion of catabolites and finally improve the appearance and tones of the skin.

Furthermore, the synergy between the laser on the adipocyte membrane and the current to make the cell more permeable, enables us to create an effect that is far superior than that obtained when using alternative applications.

### DISCLOSURE OF THE INVENTION

The technical problem that this invention overcomes refers to an increase in the effectiveness of treating cellulite. This technical problem is overcome by the device for treating cellulite that is proposed by this invention and defined in claim 1.

More specifically, the device for the treatment of cellulite relates to a quadrangular body with a plurality of support elements, at least one for every vertex of the body, wherein said elements end in a round tip, which is in contact with the skin to be treated and is **characterised in that** a plurality of laser emitters are attached to the body of that head, whereas a plurality of electrodes emitting current are housed in the support elements, at least one per support element; and wherein said electrodes define a zone on the skin, which receives the combined action of the plurality of lasers and of the currents generated by the electrodes.

In a practical embodiment, there are five laser emitters, arranged evenly in the centre of the body of the head with respect to a central diode, in the form of a cross. Furthermore, the laser diodes have a wavelength ranging from 640 to 690 nm, preferably 640 nm, in the form of a deep-red visible light that penetrates more than 15 mm into the tissues. It emits in the form of a non-spot beam.

In a practical embodiment, there are four electrodes housed inside the support element and in contact with the skin through the round tips. They are insulated and preferably emit high-voltage currents.

In a second aspect of the invention, the use of the device is claimed for the treatment of cellulite.

Thanks to the device and the method of application described, a specific combination is obtained that favours the release of this fat, generating a process that breaks down the triglycerides stored in the adipocytes into fatty acids and glycerol..

Another advantage of the invention is that it increases the permeability of the adipocyte wall, so said triglycerides can go through the pores in the membrane, and circulate in the blood and lymphatic system, being turned into fuel, thus favouring their elimination through physical exercise.

The combination of high density laser emitters (640 to 690 nm), with current emitters and a massage head, offer an effective combination for the treatment of cellulite. This innovative combination enables the emptying of adipocytes, the built-up fat to be eliminated and tissue to be toned up. This not only reduces the volume, but also shapes the treated zones.

Throughout the description and the claims, the use of the word "comprises" and its variants is not intended to exclude other technical characteristics, additives, components or steps. For the skilled in the art, other objects, advantages and characteristics of the invention will be inferred partly from the description and partly from the practice of the invention. The following examples and drawings are included by way of illustration, and are not to be interpreted as limitative of the invention as claimed. Furthermore, this invention covers all the possible combinations of specific and preferred embodiments described herein.

### BRIEF DESCRIPTION OF THE DRAWINGS

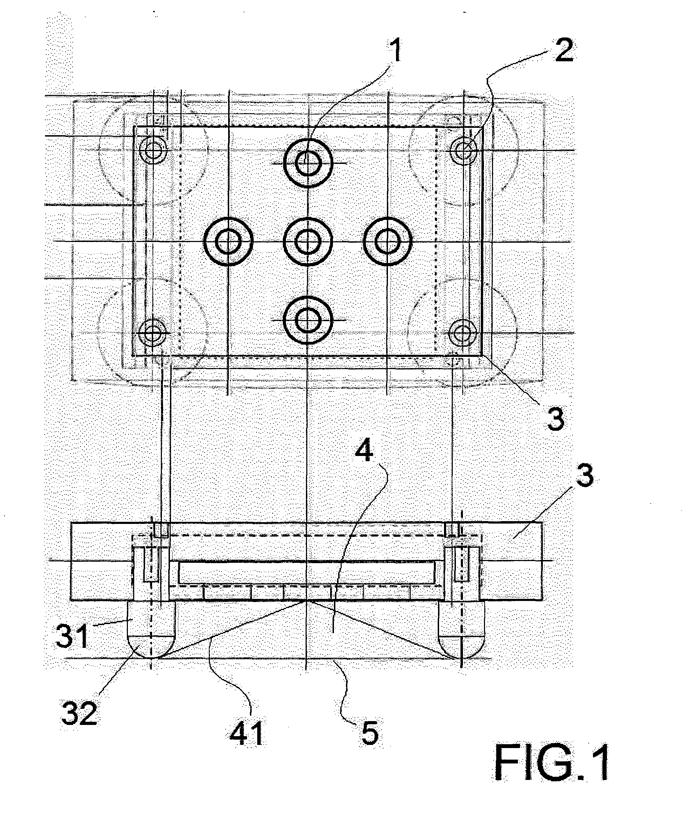

Figure1 shows a front view and plan view of the device for the treatment of cellulite, object of this invention.

### DETAILED DESCRIPTION OF AN EMBODIMENT

The device and method that is the object of this invention describes a non-invasive procedure for removing a build-up of localized fat rapidly, safely and long-lastingly. This invention basically combines the application of the invention known as Low Level Laser Therapy (LLLT) with currents of medium frequency, high voltage and low intensity.

The application of the laser stimulates the lysis of the fat (triglycerides) inside the adipocytes, producing fatty acids and glycerol, due to the stimulation that affects the mitochondria of these cells, which then produce ATP, i.e., Adenosine Triphosphate, which is a basic nucleotide in obtaining cell energy. It is composed of a nitrogenated base (adenine) attached to carbon 1 from a pentose sugar, ribose, which has three phosphate groups bonded to carbon 5. It is produced during photosynthesis and cell respiration, and is consumed by many enzymes in the catalysis of numerous chemical processes.

After the fatty acid molecules have been obtained, these having been mobilised so they can be removed, it is necessary to enable them to leave the inside of the cell, which is done by applying a current of medium frequency, high voltage and low intensity (using electrodes made of insulating material), in order to open the pores in the membranes of these cells, through which the fatty acid molecules will be removed. The current applied increases the permeability of the membrane of these cells.

The high-density laser emissions, through laser diodes whose emission ranges from 640 to 690 nm, preferably 640 nm, in the form of a deep-red visible light, penetrate more than 15 mm into the tissues. i.e., under the surface of the skin, where the cells in the organisms that are responsible for storing fat (adipocytes) are found.

The combination with current applicators, acts in a safe and selective way on the adipocyte membrane receptors and causes biochemical stimuli that favour the removal of built-up fat.

As can be seen in Figure 1, in a practical embodiment of the invention, the device comprises a plurality of laser light emitters (1) and a plurality of current emitters (2), preferably five laser light emitters (1) and four current emitters (2), plus a head or massaging element, which has not been shown.

More specifically, the emitters or laser diodes (1) are located in the centre (3) of the body of the device (3), arranged evenly in the form of a cross with respect to the central emitter, whereas the emitters or current electrodes (2) are located at the vertexes of said body of the device (3), running underneath and being housed inside a cylinder (31) with a round tip (32), defining a gap between them (4) where the emission (41) of the laser diodes (1) on the skin (5) is focused, in such a way that it is the electrodes (2) that delimit the zone where the current emitted and the laser act together.

The cylinders or support elements (31) that are located in the body of the device (3), comprise the electrodes (2), which have been made with metal lined with insulating material.

Said laser emitters and current emitters are applied for a maximum of twenty minutes, before subdermic therapy is applied with drainage through suction by fold rolling to absorb the waste generated and to carry out deep drainage, using the massage head. Furthermore, a smoothing effect of the skin is generated, which improves its appearance and tones it up.

In contrast to other state-of-the-art techniques, the procedure that is the object of this invention does not cause any damage to the adipocyte walls or to nearby tissues, it only induces the emptying of the build-ups of fat and tones up the tissues that are being treated, improving not only the vascularisation but also the cell metabolism. However, to obtain an effective treatment, the application of the device has to be supplemented with a diet that is rich in proteins and low in fats, as well as exercise to remove the lipids extracted from the adipocytes.

A description is given below of the effects of each element of the device (laser emitters, current emitters and massage head) on the body subjected to treatment.

### Laser emitters

Laser emission is a high-precision technique, and depending on its wavelength, it has different functions on the human body. The 640 nm wavelength is preferably selected because of its deep penetration into the human tissue in view of its absorption capability of red light. It is also able to stimulate the mitochondria in the adipose cells and achieve lypolisis in a natural way through the enzyme lipase, which transforms triglycerides and long-chain fats, into fatty acids and glycerol. These lighter lipids are subsequently moved out of the fat build-ups through the drainage head and used as a source of energy in everyday physical activity. This bio-stimulation is athermal, neither generating heat nor altering the protein structure.

One of its advantages is that it does not alter the adipocyte walls in a permanent way, increasing their permeability temporarily. Furthermore, it activates the lysis through natural enzyme processes (lipase), enhancing cell reactions, and turns the chains of heavy fatty acids (triglycerides) into lighter ones (fatty acids and glycerol), which are consumed naturally by the organism.

### Polar bio-currents

This is a technique for electrically stimulating the fat cells or adipocytes. Under the action of an electric field that is created, the adipocytes reactivate their exchanges and release all their contents. The frequencies emitted (ranging from 450 to 950 Hz) through the device can activate the biopolymers and cell receptors by resonance, because the communication and synchronism between them is enhanced.

These currents activate the cell's internal mechanisms, taking it to a normal state. One of them consists of aligning the bonds in the protein chains in the membrane, causing the membrane to depolarise, thus increasing its permeability, which favours the expulsion of the catabolites and any other residue coming from the combustion in the cell's internal metabolism, and receiving from the interstitial liquid, a larger amount of nutrients and oxygen, as well as activators of the energy metabolism (corticoids and T3/T4) and cell peptides that appear through energy production. All of this manages to take the cell into a state of balance, which means permanent oxidation and a reduction of the DH enzymes. This implies a constant flow of flavine electrons from the membrane; these changes are in response to the stimulus brought about by the system frequencies, and with these changes the synthesis of enzymes begins, and with this, cell phagocytosis.

Applying the device to the treatment of lipodystrophies triggers off a series of action mechanisms:
*a)* Hydrolipidic action mechanism, wherein stimulation of the neurovegetative system terminations causes the release of the cyclic AMP from inside adipocytes, giving rise to the degeneration of the triglycerides. In the cellulitic zones, the adipocytes concentrate as "static" cells; the application of currents mobilises these cells by facilitating the circulation of the lipid breakdown products and the residues from cell combustion.
*b)* Anti-inflammatory action, wherein reabsorbing metabolites brings about a reduction in the edematous reactions. The effect of the current modifies the polarisation, and with it the permeability of the cell membrane, changing the distribution of the Na+ and K+ ions, and the water content in the cells.
*c)* Vasodilation action, wherein, because the current stimulates the *nervi vasorum,* this hypervascularisation modifies the cell metabolism, enabling cells to increase their exchanges and improve their nutrition.

### Endomassage suction by rolling

Depressions and undulations are a normal feature of cellulite, owing to the grouping of the adipocytes or fat cells. They increase in size and group together in nodules with a fibrotic structure, which compresses the blood and lymphatic circuits "caged" between connective tissue fibres.

The device that is the object of this invention provides means for massage through suction that act on a hypodermic level massaging by negative pressure. Traditional massages push the tissues towards the interior of the body, whereas the endomassage by fold rolling that is carried out by the massage head comprised in the device of the invention, massages from inside to outside, which make it possible to make the fluids go from the interior to the exterior, where they are reabsorbed by the lymphatic system.

The forceful stretching of said fibres caused by the suction in the form of fold rolling, breaks these fibres and allows the homogenisation of the fatty tissue. This massage by deep suction, increases the vascularisation, the correct drainage and makes it possible to reabsorb the waste from the lypolisis and water retention, by manual displacement towards the natural elimination circuits, which favours the disappearance of the edema caused by the resistance of cellulite.

The cutaneous depressions caused by these build-ups are thus reduced, which reduces the protrusions and improves the appearance of the "orange peel skin". The physiological effects that are described are as follows:
*a)* It favours oxygenation of the various tissues on which it acts. Where striae are concerned, it improves their vascularisation, having a positive effect on their appearance.
*b)* It makes the skin suppler, toning it up.
*c)* It makes the body temperature more homogeneous through vasodilation, given that we know cellulite generates "cold zones" due to poor blood circulation.

## Claims

1. A device for the treatment of cellulite comprising means for massaging by suction, a quadrangular body (3) with a plurality of support elements (31), at least one for each vertex of the body, wherein said support elements (31) end in a rounded tip (32) that is contactable with the skin (5) to be treated, and that is **characterised in that** a plurality of laser emitters (1), which are laser diodes whose wavelength ranges from 640 to 690 nm, are housed on the quadrangular body (3), whereas a plurality of current emitter electrodes (2) made of insulating material are housed on the support elements (31), at least one per support element, and wherein said electrodes (2) define an area (4) on the skin (5) where there is a combined action of emission (41) from the plurality of lasers (1) and from the currents, which are of high voltage and low intensity in a frequency range from 450 - 950 Hz, generated by the electrodes (2).

2. A device according to claim 1 wherein there are five laser emitters (1) evenly arranged in the centre of the quadrangular body (3) with respect to a central diode, in the form of a cross.

3. A device according to the previous claims 1 and 2 wherein there are four electrodes (2) housed inside the support element (31) and contactable with the skin (5) through the rounded tips (32).

4. A device according to claim 1 wherein the laser diodes (1) have a wavelength of 640 nm, in the form of a deep-red visible light penetrating more than 15 mm into the tissues.

5. Use of the device in claims 1 to 4 for the treatment of cellulite.

## Patentansprüche

1. Vorrichtung zur Behandlung von Zellulitis, umfassend Mittel zur Saugmassage, einen viereckigen Körper (3) mit einer Vielzahl von Trägerelementen (31), mindestens eins für jeden Scheitel des Körpers, wobei diese Trägerelemente (31) in einer abgerundeten Spitze (32) enden, die mit der zu behandelnden Haut (5) in Kontakt bringbar ist, und die **dadurch gekennzeichnet ist, dass** eine Vielzahl von Laserstrahlern (1), die Laserdioden mit Wellenlängen zwischen 640 und 690 nm sind, auf dem viereckigen Körper (3) angeordnet sind, wobei eine Vielzahl von Stromemitterelektroden (2) aus Isoliermaterial auf den Trägerelementen (31) angebracht sind, wenigstens eine pro Trägerelement, und wobei diese Elektroden (2) eine Zone (4) auf der Haut (5) definieren, auf der eine kombinierte Aktion der Strahlung (41) der Vielzahl von Lasern (1), und der Ströme stattfindet, die Hochspannungsströme niederer Intensität in einem Frequenzbereich von 450 - 950 Hz sind, und von den Elektroden (2) erzeugt werden.

2. Vorrichtung gemäss Anspruch 1, wobei fünf Laserstrahler (1) gleichmässig, in Kreuzform, im Zentrum des viereckigen Körpers (3) in Bezug auf eine zentrale Diode angeordnet sind.

3. Vorrichtung gemäss der Ansprüche 1 und 2, wobei vier Elektroden (2) in dem Trägerelement (31) angeordnet sind und durch die abgerundeten Spitzen (32) mit der Haut (5) in Kontakt bringbar sind.

4. Vorrichtung gemäss Anspruch 1, wobei die Laserdioden (1) eine Wellenlänge von 640 nm haben, die in Form eines tiefroten sichtbaren Lichtes mehr als 15 mm in die Gewebe eindringen.

5. Benutzung der Vorrichtung gemäss Anspruch 1 bis 4 zur Behandlung von Zellulitis.

## Revendications

1. Dispositif pour le traitement de la cellulite, comprenant des moyens pour massage par aspiration, un corps carré (3) avec une pluralité d'éléments de support (31), au moins un pour chaque vertex du corps, ces éléments de support (31) se terminant dans un bout arrondi (32) qui peut avoir contact avec la peau (5) à traiter, et qui se **caractérise par** ce qu'une pluralité de émetteurs de laser (1), qui sont des diodes laser avec des longueurs d'onde entre 640 et 690 nm, sont logés sur le corps carré (3), de manière qu'une pluralité d'électrodes émitteur de courant (2) d'un matériel isolant sont logés sur les éléments de support (31), au moins une par élément de support, ces électrodes (2) définissant une zone (4) sur la peau (5) sur laquelle existe une action combinée d'émission (41) de la pluralité de lasers (1), et des courants, qui sont des courants de haute tension et basse intensité dans une gamme de fréquences de 450 - 950 Hz, et sont générés par les électrodes (2).

2. Dispositif suivant la revendication 1, où cinq émetteurs de laser (1) sont agencés uniformement, dans la forme d'une croix, au centre du corps carré (3) en rapport avec la diode centrale.

3. Dispositif suivant les revendications précédentes 1 et 2, où quatre électrodes (2) sont logées dans l'élément de support (31) et peuvent avoir contact avec la peau (5) par les bouts arrondis (32).

4. Dispositif suivant la revendication 1, où les diodes de laser (1) ont une longueur d'onde de 640 nm, dans la forme d'une lumière visible d'un rouge profond qui pénètre plus de 15 mm dans les tissus.

5. Emploi du dispositif suivant la revendication 1 à 4 pour traiter la cellulite.
